(19) Europäisches Patentamt / European Patent Office / Office européen des brevets

(11) **EP 3 491 703 B1**

(12) **FASCICULE DE BREVET EUROPEEN**

(45) Date de publication et mention de la délivrance du brevet:
**13.05.2020 Bulletin 2020/20**

(21) Numéro de dépôt: **17752303.2**

(22) Date de dépôt: **25.07.2017**

(51) Int Cl.:
*H01S 3/067* (2006.01)    *H01S 3/16* (2006.01)
*H01S 3/30* (2006.01)    *G01N 21/64* (2006.01)
*A61B 5/00* (2006.01)

(86) Numéro de dépôt international:
**PCT/EP2017/068710**

(87) Numéro de publication internationale:
**WO 2018/019805 (01.02.2018 Gazette 2018/05)**

(54) **SOURCE LASER FEMTOSECONDE**

FEMTOSEKUNDENLASERQUELLE

FEMTOSECOND LASER SOURCE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2016 FR 1657179**

(43) Date de publication de la demande:
**05.06.2019 Bulletin 2019/23**

(73) Titulaires:
• **Centre National de la Recherche Scientifique**
**75016 Paris (FR)**
• **Université de Limoges**
**87032 Limoges (FR)**

(72) Inventeurs:
• **FEVRIER, Sébastien**
**87110 Bosmie-L'Aiguille (FR)**
• **KOTOV, Leonid**
**87100 Limoges (FR)**
• **HIDEUR, Ammar**
**76140 Le Petit Quevilly (FR)**

(74) Mandataire: **Priori, Enrico**
**Marks & Clerk France**
**Immeuble "Visium"**
**22, avenue Aristide Briand**
**94117 Arcueil Cedex (FR)**

(56) Documents cités:
**US-A1- 2012 195 330**

• **PATRICK CADROAS ET AL: "Three-Photon Microscopy with a Monolithic All-Fiber Format Laser Emitting at 1650 nm", ADVANCED PHOTONICS 2016 (IPR, NOMA, SENSORS, NETWORKS, SPPCOM, SOF), OSA TECHNICAL DIGEST (ONLINE) (OPTICAL SOCIETY OF AMERICA, 2016), SOM4F.4, 2016, page SoM4F.4, XP055362891, DOI: 10.1364/SOF.2016.SoM4F.4 ISBN: 978-1-943580-14-9**
• **WANG KE ET AL: "Tunable high-energy soliton pulse generation from a large-mode-area fiber and its application to third harmonic generation microscopy", APPLIED PHYSICS LETTERS, A I P PUBLISHING LLC, US, vol. 99, no. 7, 15 août 2011 (2011-08-15), pages 71112-71112, XP012153608, ISSN: 0003-6951, DOI: 10.1063/1.3628337 [extrait le 2011-08-19]**
• **GAPONOV D A ET AL: "High power all-fibered femtosecond master oscillator power amplifier at 1.56 ìm", OPTICS LETTERS, OPTICAL SOCIETY OF AMERICA, vol. 37, no. 15, 1 août 2012 (2012-08-01), pages 3186-3188, XP001577512, ISSN: 0146-9592, DOI: 10.1364/OL.37.003186 [extrait le 2012-07-25]**
• **J C JASAPARA ET AL: "Simultaneous direct amplification and compression of picosecond pulses to 65-kW peak power without pulse break-up in erbium fiber References and links", OPT. EXPRESS, vol. 15, no. 26, 24 décembre 2007 (2007-12-24), pages 17494-17501, XP055362946,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- GAPONOV D ET AL: "2 m all-fiber dissipative soliton master oscillator power amplifier", SPIE - INTERNATIONAL SOCIETY FOR OPTICAL ENGINEERING. PROCEEDINGS, S P I E - INTERNATIONAL SOCIETY FOR OPTICAL ENGINEERING, US, vol. 9728, 11 mars 2016 (2016-03-11), pages 972834-972834, XP060067475, ISSN: 0277-786X, DOI: 10.1117/12.2211574 ISBN: 978-1-5106-0753-8

- D.J. RICHARDSON ET AL: "Passive, All fibre Source of 30 fs Pulses", ELECTRONICS LETTERS, vol. 28, no. 8, 9 avril 1992 (1992-04-09), XP055361982,

**Description**

**DOMAINE TECHNIQUE**

La présente description concerne une source laser femtoseconde et un procédé de génération d'impulsions laser femtosecondes. Elle concerne plus précisément une source laser femtoseconde adaptée à la microscopie multi-photonique pour l'imagerie des tissus biologiques.

**ETAT DE L'ART**

La microscopie multi-photonique permet de produire des images de tissus biologiques in-vivo ou ex-vivo et trouve des applications par exemple dans les domaines des neurosciences, de l'embryologie ou de l'oncologie. Ainsi, des microscopes multi-photoniques commerciaux utilisent l'interaction de deux photons avec les molécules d'un milieu organique pour y repérer des organelles et tracer, en collectant la fluorescence émise, une cartographie dynamique de ces organelles. Les meilleurs microscopes commerciaux permettent de visualiser les tissus à 200 $\mu$m sous la surface des tissus. En utilisant l'interaction de trois photons avec les tissus, on peut visualiser les tissus situés à plus grande profondeur (>800 $\mu$m) avec le même rapport signal sur bruit.

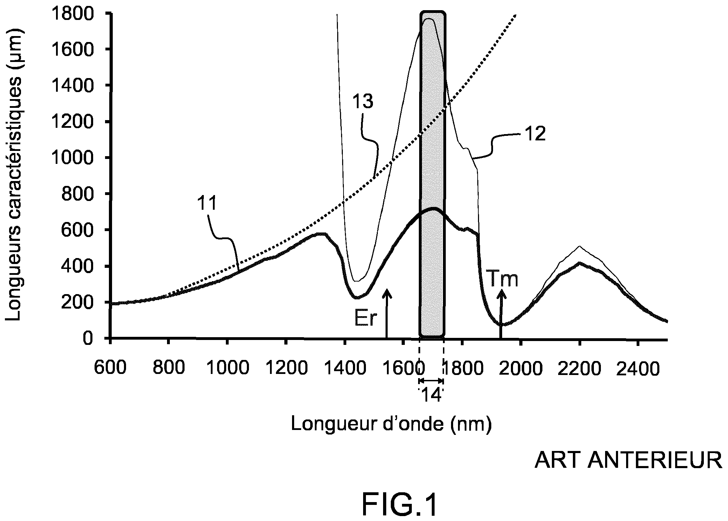

La FIG. 1 illustre différentes longueurs caractéristiques de la réponse de tissus aqueux désorganisés, comme ceux du cortex cérébral, en présence d'impulsions laser. Les longueurs caractéristiques sont fonction de la longueur d'onde des impulsions. Sur la FIG. 1, la courbe 12 représente la longueur caractéristique d'absorption ($l_a$) dans l'eau, définie comme la longueur pour laquelle la puissance optique du signal incident est atténuée d'un facteur e, où $e$ est le nombre d'Euler ; la courbe 13 représente la longueur caractéristique de diffusion ($l_d$) et la courbe 11 représente une longueur effective d'atténuation ($l_{eff}$) qui prend en compte l'absorption et la diffusion ($l_{eff}^{-1} = l_a^{-1} + l_d^{-1}$). Ces différentes courbes, et notamment la courbe 11, illustrent le fait que, dans une fenêtre 14 de longueur d'onde autour de 1675 nm (par exemple, 1675 nm +/- 25 nm), les impulsions laser pénètrent plus en profondeur dans les tissus aqueux.

La déposante a constaté plus précisément que, pour l'imagerie en profondeur dans des milieux biologiques, il faudrait idéalement, compte-tenu des propriétés des tissus aqueux désorganisés tels que les milieux biologiques, outre une longueur d'onde optimisée proche de 1675 nm, une source laser permettant la génération d'impulsions laser de forte puissance crête, typiquement de l'ordre de 100 kW ou davantage, et de durée inférieure à 200 fs. Une telle source laser couplée à un microscope permettrait d'acquérir des images en profondeur du milieu biologique avec un bon rapport signal sur bruit.

Il existe des sources lasers permettant la génération d'impulsions ultra-brèves et présentant une telle puissance crête ; ces sources laser sont constituées d'une succession d'éléments, comprenant par exemple un oscillateur-injecteur, un étireur, un ou plusieurs amplificateurs, un compresseur et un élément de conversion de longueur d'onde.

Le document intitulé « In vivo three-photon microscopy of subcortical structures within an intact mouse brain », de Horton et al., publié le 20 janvier 2013, par «Nature Photonics », décrit ainsi une technique de microscopie, applicable à l'imagerie in-vivo, notamment pour produire in-vivo des images de cerveau de souris. Des impulsions de haute énergie à une longueur d'onde de 1675 nm sont produites au moyen d'un barreau à cristal photonique par auto-décalage fréquentiel à partir d'impulsions laser source d'entrée de longueur d'onde 1550 nm. En sortie, chaque impulsion présente une puissance crête de 1 mégawatt (MW) et une durée à mi-hauteur en intensité de 114 femtosecondes. Le train d'impulsions a une cadence de 1 mégahertz (MHz), ce qui correspond à une puissance moyenne de 67 milliwatt (mW).

Cependant, dans les sources lasers connues de l'art antérieur, lorsque le faisceau laser passe d'un élément à l'autre, par l'intermédiaire de chemins en espace libre, il est nécessaire de collimater puis focaliser ce faisceau laser dans chacun des éléments, amplificateur et/ou élément de conversion de longueur d'onde, que ceux-ci soient constitués de cristaux non-linéaires ou de fibres. Ainsi, la maintenance et le réglage de telles sources lasers doit être effectuée régulièrement, ce qui peut être contraignant pour les utilisateurs de ces sources lasers, notamment lorsque ces utilisateurs ne disposent pas nécessairement du temps ou des équipements, ou même des connaissances pour effectuer une telle maintenance.

Il apparaît ainsi un besoin pour une source laser, présentant des performances adaptées pour produire des images en profondeur dans les tissus vivants et dont la maintenance soit simplifiée.

PATRICK CADROAS ET AL: "Three-Photon Microscopy with a Monolithic All-Fiber Format Laser Emitting at 1650 nm", ADVANCED PHOTONICS 2016, SOM4F.4, et le document WANG KE ET AL: "Tunable high-energy soliton pulse generation from a large-mode-area fiber and its application to third harmonie generation microscopy",APPLIED PHYSICS LETTERS, US, vol. 99, no. 7, 15 août 2011, pages 71112-71112, décrivent une source laser femtoseconde adaptée à la microscopie multi-photonique pour l'imagerie des tissus biologiques, comprenant une fibre à décalage de fréquence adaptée pour recevoir une impulsion à une première longueur d'onde et générer par auto-décalage Raman un soliton fondamental à une deuxième longueur d'onde.

# EP 3 491 703 B1

**RESUME**

La présente invention a pour objet, selon un premier aspect, une source laser femtoseconde selon la revendication 1.

Du fait de l'utilisation combinée d'une fibre amplificatrice dopée dans le même matériau que celle de l'oscillateur laser d'injection et d'une fibre à décalage de fréquence, il est possible d'obtenir un train d'impulsions à la fois ultra-brèves (de durée par exemple inférieure à 100 femtosecondes), et de forte puissance crête (supérieure par exemple à 100 kW), à cadence élevée (par exemple entre 0,1 et 100 MHz). Dans la suite de la description, on parlera d'impulsions femtosecondes pour des impulsions de durée inférieure à 100 femtosecondes.

En outre, la deuxième longueur d'onde étant fonction du matériau de dopage et de la longueur de la fibre à décalage de fréquence, on peut obtenir des impulsions à une deuxième longueur d'onde adaptée à une application donnée, par exemple aux applications d'imagerie citées en introduction.

L'utilisation et la maintenance d'une telle source laser sont simplifiées en ce que la source laser se prête à une réalisation sous forme de dispositif monolithique, dans lequel les différents composants optiques (oscillateur laser, amplificateur de puissance et fibre à décalage) sont intégrés les uns aux autres, raccordés via les fibres optiques et ne nécessitent pas de réglages. La source laser est en outre stable dans le temps et immune aux perturbations environnementales telles que vibrations mécaniques.

Dans au moins un mode de réalisation de la source laser, la fibre optique amplificatrice présente une aire modale supérieure ou égale à $200\,\lambda_1{}^2$. Une telle aire modale augmente la puissance crête de l'impulsion produite par la fibre optique amplificatrice.

Dans la source laser, la fibre optique amplificatrice présente une longueur supérieure à la distance à partir de laquelle la fibre optique amplificatrice opère en régime non-linéaire. De la sorte, des effets non-linaires tels que l'auto-modulation de phase permettent de comprimer temporellement les impulsions et d'augmenter encore la puissance crête pouvant être obtenue en sortie de la fibre amplificatrice.

Dans la source laser, la fibre à décalage de fréquence présente une aire modale supérieure à celle de la fibre amplificatrice. Une telle combinaison modale permet d'augmenter de manière notable la puissance crête de l'impulsion obtenue en sortie de la source laser.

Dans au moins un mode de réalisation de la source laser, l'oscillateur laser d'injection est un oscillateur à fibres à gestion de dispersion chromatique configuré pour générer des impulsions à dérive de fréquence. De telles impulsions présentent une importante dérive en fréquence et peuvent ainsi être amplifiées davantage par rapport à des impulsions sans dérive de fréquence.

Dans au moins un mode de réalisation de la source laser, l'oscillateur laser d'injection comprend un oscillateur solitonique délivrant des impulsions sans dérive de fréquence.

Dans au moins un mode de réalisation de la source laser, l'oscillateur solitonique est suivi d'une fibre optique à dispersion normale configurée pour étirer temporellement les impulsions générées par l'oscillateur.

Dans au moins un mode de réalisation de la source laser, le matériau de dopage est une terre rare choisie parmi l'ytterbium, le praséodyme, l'erbium, le thulium et l'holmium. Le matériau de dopage peut également être le bismuth. Ces matériaux permettent d'aboutir à des impulsions en sortie de la fibre à décalage de fréquence qui ont une longueur d'onde adaptée aux applications d'imagerie précitées.

Dans au moins un mode de réalisation de la source laser, la fibre optique amplificatrice et la fibre à décalage de fréquence sont soudées l'une à l'autre. En outre, la fibre optique de sortie de l'oscillateur laser d'injection et la fibre amplificatrice sont soudées l'une à l'autre. La source laser est ainsi monolithique et ne nécessite pas de réglage. On évite également les chemins d'air.

Dans au moins un mode de réalisation, la source laser comprenant en outre un filtre passe-haut pour éliminer des résidus d'impulsion à la première longueur d'onde $\lambda_1$. Ceci permet d'augmenter l'efficacité des impulsions produites par la source laser dans les applications d'imagerie précitées.

La présente invention a pour objet, selon un deuxième aspect, un système d'imagerie comprenant une source laser femtoseconde selon la présente invention, adaptée à l'émission d'impulsions vers un objet en profondeur dans un milieu biologique et un microscope pour former et acquérir une image de l'objet à partir de la lumière de fluorescence rétrodiffusée par l'objet.

La présente invention a pour objet, selon un troisième aspect, un procédé de génération d'impulsions laser femtosecondes selon la revendication 10.

**FIGURES**

D'autres avantages et caractéristiques de la technique présentée ci-dessus apparaîtront à la lecture de la description détaillée ci-dessous, faite par référence aux figures dans lesquelles :

- la FIG.1, déjà décrite, est un diagramme illustrant les caractéristiques de la réponse de tissus aqueux en présence d'une source laser ;
- les FIGS.2A-2B illustrent des modes de réalisation d'une source laser selon la présente description ;
- les FIGS.3A-3C illustrent des propriétés des impulsions laser générées par la source laser selon la présente description ;
- les FIGS.4A-4E illustrent différent aspects du phénomène de compression solitonique ;
- les FIGS.5A-5C illustrent différent aspects du phénomène d'auto-décalage Raman des solitons ;
- les FIGS.6A-6B illustrent des propriétés d'une source laser selon la présente description ;
- les FIGS.7A-7B illustrent des propriétés d'une source laser selon la présente description ;
- la FIG.8 est une image obtenue par un système d'imagerie selon la présente description.

## DESCRIPTION DETAILLEE

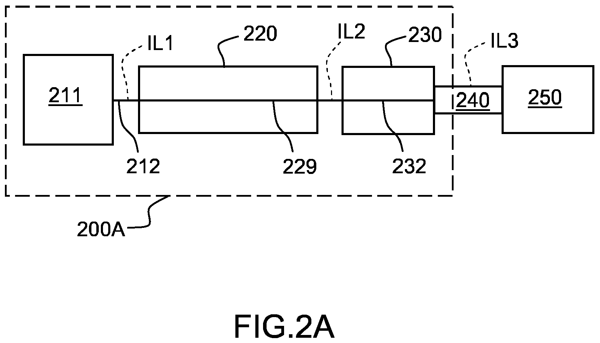

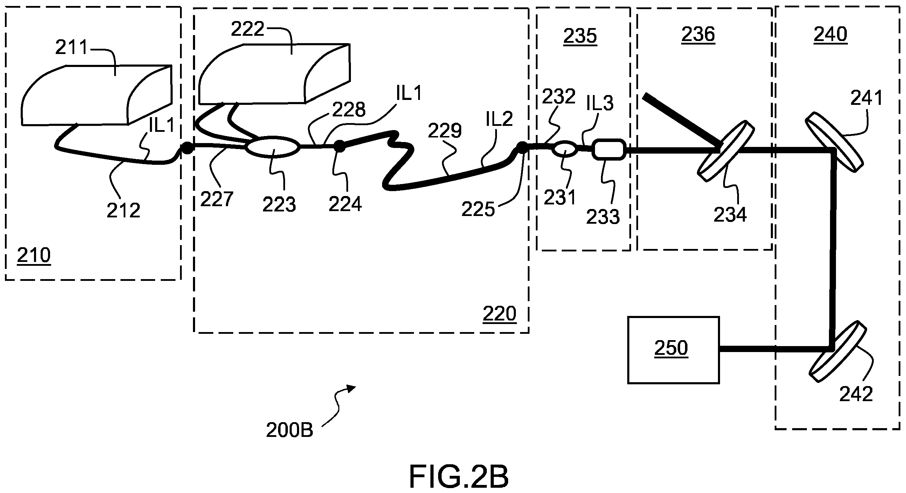

La FIG. 2A représente de façon schématique un mode de réalisation d'une source laser 200A selon la présente description, adapté par exemple à la microscopie multiphotonique et la FIG. 2B un exemple de réalisation plus détaillé d'une source laser 200B selon la présente description. La source laser 200A ou 200B constitue un dispositif de génération d'impulsions laser.

La source laser 200A représentée sur la FIG. 2A comprend trois composants principaux, à savoir :

- un oscillateur laser d'injection 211,
- un amplificateur de puissance 220 à fibre optique 229, et
- un dispositif 230 à décalage de fréquence, comprenant une fibre optique 232.

La source laser 200A génère un train d'impulsions optiques qui sont acheminées, au moyen d'un composant optique 240 de sortie, vers un microscope 250 permettant la formation et l'acquisition d'images.

L'oscillateur laser d'injection 211 comprend au moins une fibre optique dopée dans un matériau donné. La fibre optique dopée de l'oscillateur laser d'injection 211 est par exemple une fibre optique constituée d'un matériau luminescent donné (verre ou matrice vitreuse), dopé dans un matériau donné. Le matériau de dopage est un matériau optiquement actif, c'est-à-dire que, sous excitation (par exemple par des lasers de pompe internes à l'oscillateur laser 211), ce matériau émet une lumière cohérente à une longueur d'onde donnée. Dans un ou plusieurs modes de réalisation, cette fibre optique dopée est une fibre amplificatrice interne à l'oscillateur laser d'injection 211. Dans un ou plusieurs modes de réalisation, le matériau de dopage est un ion, par exemple un ion de terre rare. La terre rare est par exemple le néodyme (de symbole chimique Nd), l'ytterbium (de symbole chimique Yb), le praséodyme (de symbole chimique Pr), l'erbium (de symbole chimique Er), le thulium (de symbole chimique Tm), l'holmium (de symbole chimique Ho), ou tout autre élément fluorescent soluble dans la matrice vitreuse constituant la fibre, comme par exemple le bismuth (de symbole chimique Bi).

L'oscillateur laser d'injection 211 produit en sortie, via une fibre optique 212 de sortie, un premier train d'impulsions laser IL1, à une première longueur d'onde $\lambda_1$. Les impulsions laser IL1 sont des impulsions picosecondes (ps). Dans le cadre de la présente description, une impulsion picoseconde est une impulsion de durée comprise entre 1 et 100 ps. La cadence des impulsions laser IL1 est comprise par exemple entre 0,1 et 100 MHz.

Dans au moins un mode de réalisation de la source laser 200A, l'oscillateur laser d'injection 211 est un oscillateur laser fibré à verrouillage de modes en phase. Un tel verrouillage des modes longitudinaux de l'oscillateur laser d'injection permet d'obtenir des impulsions picoseconde. D'autres types de lasers, par exemple un laser de type « gain switch » permettent également d'obtenir des impulsions picoseconde.

La première longueur d'onde $\lambda_1$ dépend du matériau de dopage de la fibre optique dopée de l'oscillateur laser d'injection 211. Lorsque le matériau de dopage est l'erbium, la première longueur d'onde $\lambda_1$ est d'environ 1555 nm. Plus généralement, selon le matériau de dopage choisi, la longueur d'onde $\lambda_1$ peut être comprise entre 900 et 2200 nm.

L'amplificateur de puissance 220 génère, à partir du premier train d'impulsions laser IL1, un deuxième train d'impulsions laser IL2, à la première longueur d'onde $\lambda_1$. Ces impulsions laser IL2 présentent une énergie amplifiée par rapport aux impulsions laser IL1.

La fibre optique amplificatrice 229 de l'amplificateur de puissance 220 est dopée dans le même matériau que la fibre optique dopée de l'oscillateur laser d'injection 211 de sorte que la fonction de transfert représentant le gain de l'amplificateur soit adaptée au spectre de l'impulsion produite dans l'oscillateur et préserver ainsi les composantes spectrales de cette impulsion.

Au sein de la fibre optique amplificatrice 229, l'énergie de l'impulsion IL2 croît exponentiellement avec la distance de propagation au sein de la fibre optique amplificatrice 229, mais la longueur d'onde centrale de l'impulsion IL2 ne varie pas.

Dans au moins un mode de réalisation, la longueur de la fibre optique amplificatrice 229 est choisie de sorte à être supérieure à la distance à partir de laquelle la fibre optique amplificatrice 229 opère en régime non-linéaire. Outre l'effet d'amplification, la fibre optique amplificatrice 229 a sur le premier train d'impulsions laser IL1 des effets non linéaires, c'est-à-dire induisant des modifications non-linéaires (déformations, introduction de dissymétries...) du spectre en fréquence des impulsions IL1. Ces effets non linéaires sont détectables par comparaison du spectre en fréquence des impulsions IL2 par rapport au spectre en fréquence des impulsions IL1 en entrée de la fibre optique amplificatrice 229. Ces effets non-linéaires incluent par exemple l'auto-modulation de phase et la diffusion Raman stimulée.

Ainsi, lorsque la puissance crête (i.e. le rapport entre l'énergie E et $T_{FWHM}$ la durée à mi-hauteur de l'impulsion ou « full-width at half maximum », selon la terminologie anglo-saxonne) croît et atteint un seuil, le spectre de l'impulsion s'élargit par un phénomène non-linéaire d'auto-modulation de phase. En conséquence, l'impulsion est comprimée temporellement, c'est-à-dire que sa durée est réduite. La puissance crête croît alors de plus en plus vite, ce qui amplifie l'effet de l'auto-modulation de phase. Ceci permet d'obtenir une impulsion amplifiée IL2 à forte énergie (>100 nJ), et de durée réduite par rapport à une impulsion d'entrée IL1.

Dans au moins un mode de réalisation, la fibre optique amplificatrice 229 est ainsi utilisée dans un régime non-linéaire dans lequel le phénomène d'auto-modulation de phase dans un régime de dispersion anormal ($\beta_2 < 0$, $\beta_2$ étant la dispersion de vitesse de groupe exprimée en $ps^2/m$) conduit à l'obtention d'impulsions IL2 comprimées temporellement (par exemple, à une durée égale ou inférieure à la moitié de la durée de l'impulsion IL1) par rapport aux impulsions IL1 issues de l'oscillateur laser d'injection 211. Cette compression temporelle est obtenue dans la fibre optique amplificatrice 229 tant que le point de compression solitonique où l'impulsion fissionne n'est pas atteint.

Compte tenu de l'amplification, l'énergie des impulsions amplifiées dans la fibre optique amplificatrice 229 correspond à un soliton d'ordre N déterminé par la formule suivante :

$$N^2 = 2\pi \, E_2 \, T_0 \, n_2 \, / \, (|\beta_2| \, \lambda_1 \, A_{eff}) \qquad\qquad Eq.1$$

où $E_2$ représente l'énergie de l'impulsion amplifiée IL2, $T_0$ la durée de l'impulsion définie par $T_0 = T_{FWHM} / (1 + 2 \ln(1 + 2^{1/2}))$, $n_2$ l'indice non-linéaire de Kerr et $A_{eff}$ l'aire modale effective de la fibre optique. Un soliton d'ordre élevé est instable et fissionne en N solitons d'ordre 1 d'énergie inférieure (donnée par l'équation Eq.1 en prenant N = 1) en raison d'effets perturbateurs d'ordre supérieur dont font partie la diffusion Raman stimulée, l'auto-raidissement et les ordres élevés de dispersion chromatique.

Dans au moins un mode de réalisation, la longueur de la fibre optique amplificatrice 229 est en outre strictement inférieure à la distance du point de compression solitonique maximale de sorte que les effets perturbateurs d'ordre élevé, qui deviennent importants lorsque le spectre de l'impulsion s'élargit, ne provoquent pas la fission de l'impulsion IL2 dans la fibre amplificatrice 229.

Dans au moins un mode de réalisation, la fibre optique amplificatrice 229 de l'amplificateur de puissance 220 présente une très grande aire modale effective $A_{eff}$, supérieure à 200 $\lambda_1^2$, par exemple supérieure à 500 $\mu m^2$ dans le cas d'une fibre dopée à l'erbium. Par exemple, lorsque la fibre optique amplificatrice 229 est une fibre monomode, l'énergie maximale que peuvent prendre les impulsions laser IL2 amplifiées augmente avec l'aire modale effective du mode de propagation de la fibre optique amplificatrice 229. Comme la puissance crête est proportionnelle à l'énergie pour une durée d'impulsion fixée, plus l'aire modale effective est grande, plus la puissance crête des impulsions amplifiées est élevée. Dans une fibre amplificatrice à grande aire modale effective, il est possible d'atteindre une très forte énergie avant que le phénomène d'auto-modulation de phase n'impacte le spectre des impulsions amplifiées (par exemple par élargissement, déformation et/ou rupture de symétrie du spectre en fréquence) d'une manière telle que l'impulsion sera sujette aux effets perturbateurs et à la fission. La distance du point de compression solitonique correspond ainsi à la distance de propagation à partir de laquelle ces effets indésirables se produisent. Cette distance du point de compression solitonique est ainsi fonction de l'aire modale effective de la fibre optique amplificatrice 229 et de la puissance crête de l'impulsion en entrée de la fibre amplificatrice.

Le dispositif 230 à décalage de fréquence reçoit le deuxième train d'impulsions laser IL2 et génère un troisième train d'impulsions IL3. Plus précisément, pour chaque impulsion du deuxième train d'impulsions laser IL2, un soliton fondamental à une deuxième longueur d'onde $\lambda_2$ strictement supérieure à la première longueur d'onde $\lambda_1$, est généré par fission des impulsions IL2 amplifiées puis par auto-décalage Raman. L'énergie des impulsions à la longueur d'onde IL2 est donnée par l'équation Eq.1 où N = 1. La fibre 232 à décalage de fréquence est optimisée en aire effective modale pour que l'énergie de l'impulsion IL3 soit maximale.

La longueur d'onde $\lambda_2$ des impulsions IL3 est strictement supérieure à la première longueur d'onde $\lambda_1$ des deuxièmes impulsions à partir desquelles elles sont générées du fait que la diffusion Raman stimulée, qui est à l'origine de l'auto-décalage fréquentiel des solitons dans la fibre à décalage de fréquence 232, a un effet dissipatif en termes d'énergie lumineuse. En vertu du principe de conservation de l'énergie totale, la longueur d'onde $\lambda_2$ ne peut pas être inférieure à la longueur d'onde initiale $\lambda_1$.

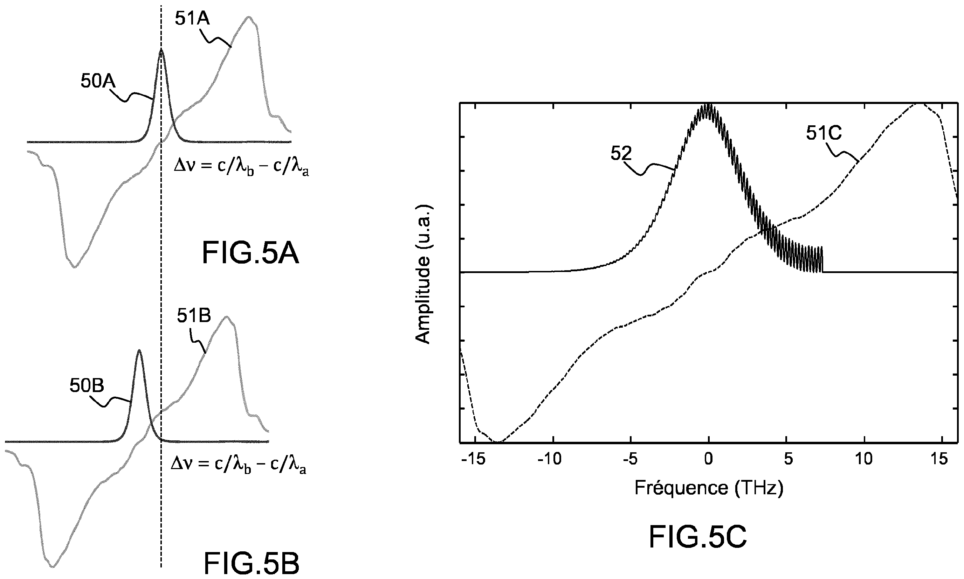

La deuxième longueur d'onde $\lambda_2$ est donc à la fois fonction du matériau de dopage de la fibre amplificatrice 229, qui détermine la première longueur d'onde $\lambda_1$, et de la diffusion Raman au sein de la fibre 232 à décalage de fréquence. La diffusion Raman ajoute à la longueur d'onde $\lambda_1$ une contribution $\delta\lambda = -\lambda_1^2/c\ \delta f$ où $\delta f$ est négatif et fonction de la susceptibilité Raman (voir les FIGS.5A et 5B) et où c est la célérité de la lumière dans le vide. Ainsi, $\lambda_2 = \lambda_1 + (\lambda_1^2/c|\delta f|)$.

La deuxième longueur d'onde $\lambda_2$ est également fonction de la longueur de la fibre à décalage de fréquence 232 et de la puissance crête de l'impulsion IL2 en entrée de la fibre à décalage de fréquence 232. Plus la fibre à décalage de fréquence 232 est longue, plus important est le décalage de fréquence subi par l'impulsion IL2 dans cette fibre. A longueur de fibre fixe, plus la puissance crête de l'impulsion IL2 est élevée, plus important est le décalage de fréquence subi par l'impulsion IL2 dans cette fibre à décalage de fréquence 232.

Dans au moins un mode de réalisation, les paramètres modaux de la fibre à décalage de fréquence 232, incluant l'aire effective modale et la dispersion chromatique, sont configurés afin de générer au moins un soliton fondamental IL3 (N = 1) à partir d'une impulsion IL2 (avec N > 1) en sortie de l'amplificateur tout en maximisant la puissance crête du soliton fondamental IL3. De plus, la longueur de la fibre 232 est choisie afin d'accorder la longueur d'onde centrale de l'impulsion solitonique IL3. Selon l'équation (1), l'énergie E3 de l'impulsion solitonique IL3 générée dans la fibre à décalage de fréquence 232 sera maximale si l'aire modale et/ou la dispersion chromatique de la fibre sont maximisées. Une forte dispersion chromatique contribue à étaler l'impulsion et, bien qu'elle présente alors une forte énergie, sa puissance crête n'est pas suffisamment accrue. La fibre à décalage de fréquence 232 présente donc une très grande aire modale, qui peut être supérieure à celle de la fibre optique amplificatrice 229, par exemple supérieure à 500 $\mu$m2, de manière à recevoir une grande partie de l'énergie de la deuxième impulsion laser IL2.

Dans au moins un mode de réalisation, la fibre optique amplificatrice 229 est soudée par un joint à la fibre à décalage de fréquence 232. Toute méthode connue de soudure de fibre optique est applicable pour réaliser cette soudure. En particulier, une soudure par fusion par arc électrique est utilisable.

Dans au moins un mode de réalisation, la fibre amplificatrice 229 et la fibre à décalage de fréquence 232 sont différentes, mais adaptées l'une à l'autre en termes d'aire modale et/ou de géométrie transverse et/ou de matériau de sorte à éviter les pertes de puissance aux soudures entre ces fibres et ainsi préserver l'énergie de l'impulsion en entrée de la fibre à décalage de fréquence 232. L'optimisation d'un joint entre fibres dissymétriques est décrite plus en détail par exemple dans l'ouvrage intitulé « Single-mode fiber optics », de Luc Jeunhomme, Chapitre 3, p. 99, éditions Marcel Dekker, New York (1983) ISBN 0-8247-7020-X.

Dans au moins un mode de réalisation, la fibre amplificatrice 229 et la fibre à décalage de fréquence 232 sont différentes et dissymétriques en termes d'aire modale, de diamètre de cœur, de différence d'indice cœur-gaine et/ou de diamètre externe. Le rendement en énergie du raccord entre ces deux fibres optiques dissymétriques est dans ce cas optimisé en réalisant une transition adiabatique, c'est-à-dire sans pertes d'énergie. Une transition adiabatique consiste en un effilement local de la fibre de plus grand cœur afin d'adapter les aires modales des deux fibres à raccorder.

Dans au moins un mode de réalisation, la fibre optique 229 amplificatrice est effilée sur tout ou partie de sa longueur pour augmenter significativement le diamètre du cœur (par exemple dans un rapport de 1 à 3 ou plus entre le début et la fin de la fibre effilée), et ainsi augmenter significativement l'énergie que peut véhiculer l'impulsion IL2 en comparaison d'une fibre à diamètre de cœur constant le long de la longueur (invariant de translation).

Dans au moins un mode de réalisation, la fibre à décalage de fréquence 232 est une fibre effilée dont l'étendue géométrique d'entrée est adaptée à celle de la sortie de la fibre amplificatrice 229.

La FIG. 2B illustre un mode de réalisation particulier d'une source laser 200B selon la présente description.

La source laser 200B représentée sur la FIG. 2B comprend trois composants principaux, un oscillateur laser d'injection 211 à fibre optique (non représentée), un amplificateur de puissance 220 à fibre optique 229, et un dispositif 230 à décalage de fréquence comprenant une fibre optique 232. Ces composants sont par exemple ceux décrits par référence à la FIG. 2A.

La fibre optique 212 en sortie de l'oscillateur laser d'injection 211 achemine les impulsions laser IL1 vers l'amplificateur de puissance 220.

Dans le mode de réalisation illustré à la FIG. 2B, l'amplificateur de puissance 220 comprend une fibre optique d'entrée 227, des lasers de pompe 222, un combineur de pompe multimode 223 (selon la terminologie anglo-saxonne, « multimode pump combiner, MPC »), une fibre optique 228 en sortie du combineur de pompe multimode 223, et une fibre optique amplificatrice 229.

La fibre optique d'entrée 227 reçoit via la fibre optique 212 les impulsions laser IL1 générées par l'oscillateur laser d'injection 211.

Les lasers de pompe 222 sont choisis en fonction de la longueur d'onde du rayonnement infrarouge multimode spatial que ces lasers de pompe 222 produisent, cette longueur d'onde étant adaptée pour produire une inversion des populations en ions de terre rare et ainsi, selon le principe de l'émission stimulée, permettre l'amplification des impulsions lasers IL1. Par exemple, dans le cas où l'ion de terre rare est l'erbium, la longueur d'onde du rayonnement issu des lasers de pompe peut être de 979 +/-3 nm ou 1532+/-3 nm.

Le combineur de pompe multimode 223 est adapté pour combiner dans la fibre monomode à double gaine 228 le rayonnement issu des lasers de pompe 222 et les impulsions laser IL1 issues de la fibre de sortie 212 de l'oscillateur laser d'injection 211. Un fond continu est ainsi ajouté aux impulsions IL1. Ces impulsions pré-amplifiées IL1 sont transmises via la fibre optique 228 dans la fibre amplificatrice 229. De manière identique à ce qui a été décrit par référence à la FIG.2A pour la génération des impulsions IL2 à partir des impulsions IL1, la fibre amplificatrice 229 génèrent des impulsions amplifiées IL2 à partir des impulsions laser IL1.

Dans le mode de réalisation illustré à la FIG. 2B, le dispositif 230 de décalage de fréquence de la FIG.2A comprend une partie 235 fibrée et une partie 236 en espace libre. La partie 235 fibrée comprend une fibre à décalage de fréquence 232. La partie fibrée peut comprendre en outre un nettoyeur de gaine 231 (selon la terminologie anglosaxonne « cladding light stripper ») et un collimateur 233. La partie 236 en espace libre comprend par exemple un filtre 234.

Le nettoyeur de gaine 231 est configuré pour supprimer tout rayonnement parasite résiduel issu des lasers de pompe 222 et non-absorbé dans la fibre amplificatrice 229. Le filtre 234 est un filtre passe-haut, configuré pour éliminer, dans l'onde optique produite par la fibre à décalage de fréquence 232, des résidus d'impulsion IL2 à la première longueur d'onde $\lambda_1$. Le collimateur 233 sert à collimater l'onde optique de sortie sur le filtre 234.

Le composant optique 240 de sortie comprend un ou plusieurs miroirs 241, 242 pour acheminer en espace libre les impulsions optiques en sortie du filtre 234 sur le microscope 250.

La fibre optique 228 est par exemple soudée par un joint 224 à la fibre optique amplificatrice 229. La fibre optique amplificatrice 229 est également soudée par un joint 225 à la fibre à décalage de fréquence 232. Toute méthode connue de soudure de fibre optique est applicable pour réaliser ces soudures. En particulier, une soudure par fusion par arc électrique est utilisable.

Dans au moins un mode de réalisation de la source laser 200A ou 200B, l'oscillateur laser d'injection 211 est un oscillateur qui comprend, outre la fibre amplificatrice interne dopée décrite par référence à la fig. 2A, des fibres à gestion de dispersion chromatique qui génèrent des impulsions à dérive de fréquence, appelées « *dispersion-managed solitons* » (DMS) dans la terminologie anglo-saxonne. Les impulsions laser générées par un tel oscillateur à fibres à gestion de dispersion chromatique sont fortement étirées temporellement en raison de la forte dispersion de vitesse de groupe de l'oscillateur laser d'injection 211 et présentent un profil temporel quasi-gaussien. Ces impulsions présentent en outre un profil spectral large et quasi-gaussien. Ces impulsions ne sont ainsi pas en limite de Fourier ($T_{FWHM}$ x $\Delta v$ > 0,44 pour des impulsions quasi-gaussiennes) et présentent ainsi une forte dérive de fréquence qui pourra être compensée (i.e. réduite, voire annulée) lors de la compression non-linéaire dans la fibre amplificatrice 229. Les impulsions issues de l'oscillateur à gestion de dispersion chromatique ont une durée limite théorique sub-300 fs, à la longueur d'onde $\lambda_1$, de sorte à pouvoir être comprimées à une durée sub-picoseconde, puis donner lieu à une impulsion IL3 de haute énergie. Comme l'énergie d'une impulsion à dérive de fréquence à spectre large peut être amplifiée à des niveaux plus élevés que ceux obtenus pour une impulsion sans dérive de fréquence, il est possible, après amplification et compression dans la fibre amplificatrice 229, d'obtenir une puissance crête élevée, par exemple en augmentant l'aire modale effective de la fibre 232 à décalage de fréquence. Ceci permettra d'obtenir un décalage fréquentiel efficace vers une autre longueur d'onde par auto-décalage Raman avec une longueur de fibre à décalage de fréquence 232 réduite et une énergie par impulsion plus élevée. Dans le cas d'un oscillateur laser d'injection 211 à fibres à gestion de dispersion chromatique, la deuxième longueur d'onde $\lambda_2$ est également fonction des longueurs des fibres à gestion de dispersion de l'oscillateur laser d'injection 211.

Dans le cas d'un oscillateur à fibres à gestion de dispersion chromatique, la longueur de la fibre amplificatrice 229 peut être choisie de sorte qu'il y ait une compression temporelle des impulsions par compensation de la dérive de fréquence des impulsions IL1 due, d'une part, à la dispersion chromatique anormale de la fibre amplificatrice 229 et, d'autre part, à l'auto-modulation de phase due à l'effet Kerr.

Dans au moins un mode de réalisation de la source laser 200A ou 200B, l'oscillateur génère des solitons, de forme temporelle et spectrale en sécante hyperbolique. Ces solitons sont en limite de Fourier (« transform-limited », selon la terminologie anglo-saxonne), caractérisés par $T_{FWHM} \times \Delta v$ = 0,31, c'est-à-dire que ce sont les impulsions les plus brèves qu'il est possible de générer avec un profil d'intensité spectral donné et qu'elles ne peuvent pas être comprimées à une durée inférieure à leur durée initiale.

Dans au moins un mode de réalisation de la source laser 200A ou 200B, l'oscillateur laser d'injection 211 est un oscillateur générant des impulsions sans dérive de fréquence, par exemple un oscillateur solitonique générant des solitons. Dans au moins un mode de réalisation, cet oscillateur solitonique est suivi d'une fibre optique à dispersion normale étirant temporellement les impulsions générées par l'oscillateur laser d'injection 211 jusqu'à une durée picoseconde. Cet assemblage produit des impulsions à dérive de fréquence similaires à celles issues d'un oscillateur à gestion de dispersion chromatique.

Dans au moins un mode de réalisation de la source laser 200A ou 200B, la fibre à décalage de fréquence 232 est une fibre sélectivement absorbante de sorte à absorber, dans l'onde optique produite dans la fibre à décalage de fréquence 232, des résidus d'impulsion à la première longueur d'onde $\lambda_1$. Dans le cas de la source laser 200B, le filtre passe-haut 234 et le collimateur 233 peuvent être supprimés.

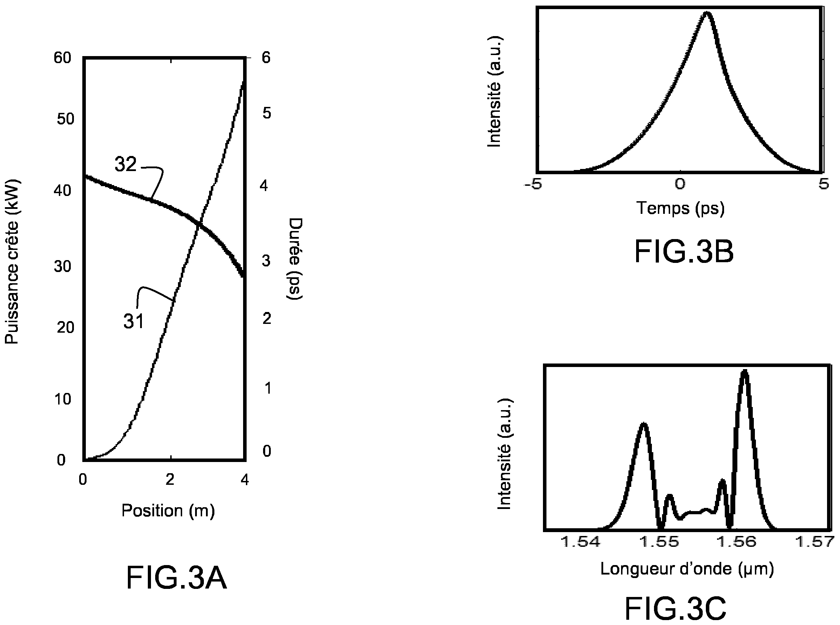

Le choix de la longueur de la fibre amplificatrice 229 est illustré par la FIG. 3A qui représente la durée (courbe 32) de l'impulsion laser IL1 et la puissance crête (courbe 31) de l'impulsion laser IL1 dans la fibre optique amplificatrice 229 de l'amplificateur de puissance 220 en fonction de la distance de propagation par rapport au point d'entrée de cette fibre optique amplificatrice 229. On observe sur cet exemple la réduction de la durée de l'impulsion laser qui intervient lorsque l'énergie des impulsions croît. La durée de l'impulsion laser diminue brutalement à partir d'environ 3 m du point d'entrée de la fibre optique amplificatrice 229 ce qui est le signe que l'auto-modulation de phase devient prédominante dans le processus de compression temporelle. L'énergie accumulée par l'impulsion laser IL2 en sortie de la fibre 229 atteint plusieurs dizaines de nJ (environ 157 nJ, soit une puissance crête de 57 kW dans l'exemple de la FIG. 3A) pour une durée à mi-hauteur de 3 ps.

La FIG. 3B représente la variation temporelle de l'intensité de l'impulsion obtenue à 3 m du point d'entrée de la fibre optique amplificatrice 229 et la FIG. 3C représente le spectre de cette même impulsion. Bien que l'impulsion soit encore intègre comme le montre la FIG. 3B, son spectre est déformé sous l'effet de l'auto-modulation de phase comme le montre la FIG. 3C. Au-delà de ce point, l'impulsion laser IL1 fissionne en plusieurs ondes solitoniques élémentaires sous les effets perturbateurs précités. La FIG. 3C est ainsi un exemple illustratif d'une déformation limite sur le spectre en fréquence, au-delà de laquelle l'impulsion perd son intégrité, voire fissionne. On observe sur cette FIG. 3C un effet d'étalement du spectre ainsi qu'une perte de symétrie entre hautes et basses fréquences alors que le spectre de l'impulsion à l'entrée de la fibre optique amplificatrice 229 est quasi-gaussien.

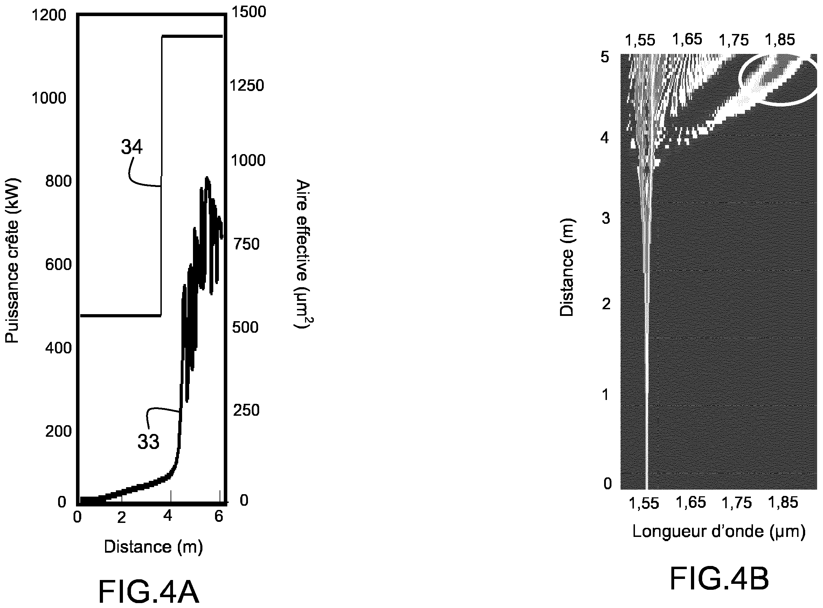

Le diagramme de la FIG. 4A illustre la manière dont l'aire modale effective (courbe 34) de la fibre amplificatrice 229 et la fibre à décalage de fréquence 232 varie, le saut de la courbe 34 correspondant au passage (à une distance de propagation de 4 m environ) de la fibre amplificatrice 229 à la fibre à décalage de fréquence 232. Le diagramme de la FIG. 4A illustre également la manière dont la puissance crête (courbe 33) de l'impulsion laser IL2 augmente dans la fibre amplificatrice 229 puis dans la fibre à décalage de fréquence 232 sous l'effet de la compression multi-solitonique. On observe que, jusqu'à une distance de propagation de 4 m par rapport au point d'entrée de la fibre 229, la puissance crête augmente quasi-linéairement (comme dans la FIG. 3A). Entre le début de la fibre à décalage de fréquence 232 et jusqu'à approximativement une distance de propagation de 0,5 m par rapport au point d'entrée (à une distance de 4 m environ) de cette fibre à décalage de fréquence 232, la puissance crête augmente exponentiellement jusqu'à une valeur de 500 kW, ce qui traduit le phénomène de compression multi-solitonique. La fibre à décalage de fréquence 232 est conçue de manière à obtenir une valeur de l'ordre N du soliton proche de l'unité et ainsi minimiser le nombre de solitons après fission de l'impulsion IL2. L'ordre N atteint ici la valeur de 2,6 ($E_2$ = 157 nJ, $T_{FWHM}$= 55 fs, $A_{eff}$ = 1250 $\mu m^2$, $\beta_2$ = 2,9 $10^{-26}$ $s^2$/m), par exemple en maximisant son aire effective modale. Puis, après 0,5 m par rapport au point d'entrée de la fibre à décalage de fréquence 232, l'impulsion IL2 fissionne en deux solitons fondamentaux dont l'impulsion solitonique IL3, qui correspond à la partie cerclée en haut à droite de la figure 4B.

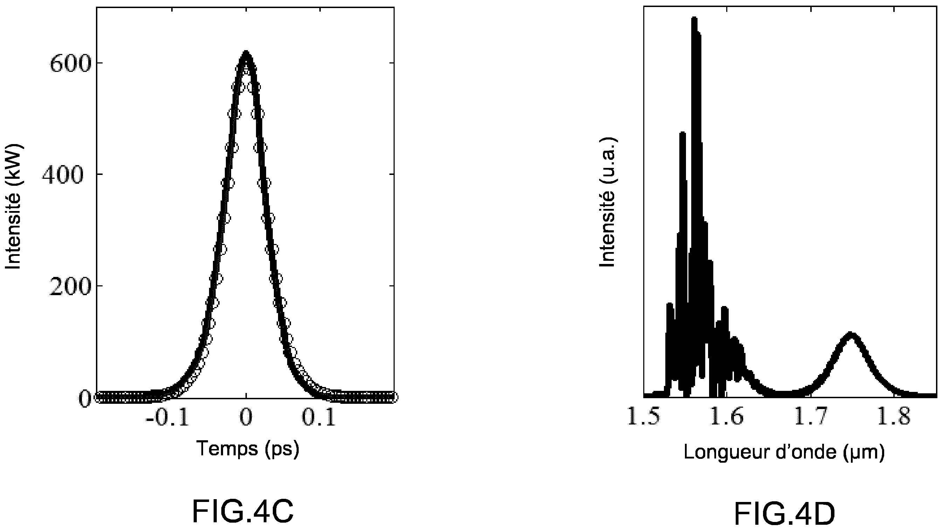

La FIG. 4C représente la variation temporelle de l'intensité de l'impulsion IL3 en sortie de la fibre 232 à décalage de fréquence. On observe que l'impulsion a une durée à mi-hauteur ultra-brève, inférieure à 100 fs. En sortie de la fibre à décalage de fréquence 232 la puissance crête de l'impulsion solitonique IL3 peut être déterminée et vaut ici 600 kW pour une durée à mi-hauteur de 100 fs. Les cercles sur la courbe de la FIG. 4C correspondent à des points d'une courbe d'ajustement (en l'occurrence, une sécante hyperbolique au carré) montrant que l'impulsion correspond bien à un soliton. La courbe de la FIG. 4D représente le spectre en sortie de fibre à décalage de fréquence 232. La longueur d'onde centrale de l'impulsion solitonique IL3 est de 1,75 $\mu$m.

Le phénomène d'auto-décalage Raman est illustré par les FIGs 5A-5C. La FIG. 5A montre le spectre en fréquence 50A d'une impulsion laser avant auto-décalage en corrélation avec la courbe 51A de la partie imaginaire de la susceptibilité Raman, et la FIG. 5B montre le spectre en fréquence 50B d'une impulsion laser après auto-décalage, en corrélation avec la courbe 51B de la partie imaginaire de la susceptibilité Raman. Une impulsion ultra-brève se propageant dans un milieu matériel est sujette à la diffusion Raman stimulée. De manière connue, l'effet Raman provoque une dérive continue de la fréquence centrale de l'impulsion par un échange d'énergie avec les phonons du milieu que forme le cœur de la fibre optique. Au travers de cette interaction lumière-matière inélastique un photon de longueur d'onde $\lambda_a$, c'est-à-dire d'énergie $E_a$ = hc/$\lambda_a$, où h est le quantum d'action ou constante de Planck (h = 6,62.$10^{-34}$ J.s) et c est la célérité de la lumière dans le vide, est absorbé par le milieu matériel. Un second photon à une énergie inférieure $E_b < E_a$ est émis par le milieu Raman à une longueur d'onde plus grande $\lambda_b > \lambda_a$. La différence d'énergie, ou défaut quantique, est transmise au milieu matériel sous la forme d'une particule correspondant à une vibration acoustique du milieu matériel, ou phonon. La partie imaginaire de la susceptibilité Raman est représentée sur la FIG. 5A, dans le cas d'exemple de la silice en fonction du décalage fréquentiel entre les deux photons mis en jeu $\Delta v = c/c\lambda_b$ -c/$\lambda_a$). Lors de l'interaction au travers de l'effet Raman entre le milieu matériel et une impulsion ultra-brève de spectre fréquentiel large, comme celle représentée aussi sur la FIG. 5A, la partie du spectre de l'impulsion pour $\Delta v > 0$ est absorbée tandis que la partie du spectre de l'impulsion à $\Delta v < 0$ est amplifiée. Cela correspond à un glissement du spectre de l'impulsion vers les basses fréquences, comme représenté sur la FIG. 5B, c'est-à-dire à un décalage du centre de l'impulsion vers les hautes longueurs d'onde.

La FIG. 5C représente de manière plus précise le spectre (courbe 52) en fréquence d'une impulsion ultra-brève se propageant dans un milieu Raman en corrélation avec la courbe 51C de la partie imaginaire de la susceptibilité Raman. L'origine des fréquences (v = 0) est prise par convention au sommet du spectre en fréquence de l'impulsion. Avec cette convention, la partie imaginaire du spectre de la susceptibilité Raman est centrée sur le spectre de l'impulsion. La partie positive du spectre de la susceptibilité Raman correspond à l'absorption par le matériau tandis que la partie négative du spectre de la susceptibilité Raman correspond à une émission. On observe que les deux spectres se superposent et que la partie de l'impulsion aux fréquences positives peut être absorbée par le milieu Raman. Dans cet exemple, le maximum d'absorption se situe à 13.2THz (courbe 51C) et la largeur à mi-hauteur du spectre (courbe 52) de cette impulsion est de l'ordre de 6 THz. Bien que le maximum du spectre en fréquence ne soit pas superposé avec le maximum d'absorption et malgré une largeur du spectre en fréquence relativement peu importante, le décalage en fréquence se produit néanmoins par diffusion Raman. Les photons à ces fréquences vont donc donner lieu à l'émission de photons à des fréquences négatives par rapport au centre de l'impulsion. Ce transfert d'énergie des fréquences positives de l'impulsion vers les fréquences négatives de l'impulsion correspond à un glissement en fréquence du centre de l'impulsion, c'est-à-dire à un décalage vers les hautes longueurs d'onde du sommet du spectre en fréquence de l'impulsion.

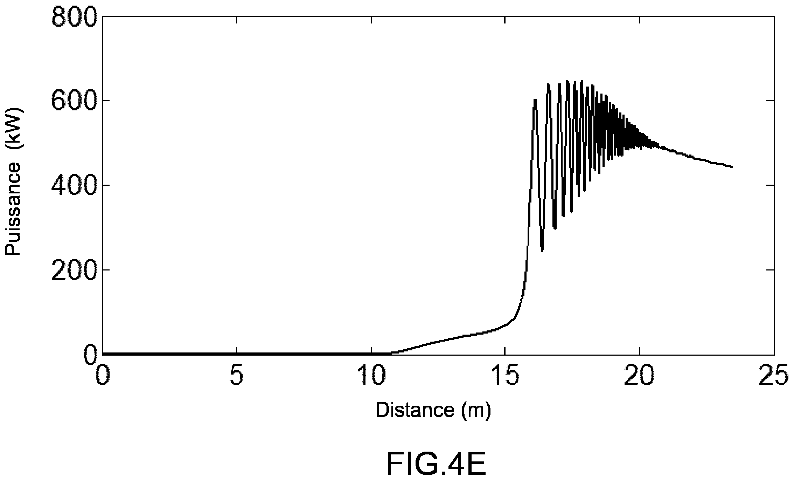

Lors de ce processus d'auto-décalage Raman, le défaut quantique contribue à une diminution de la puissance crête de l'impulsion comme observé sur la FIG. 4E. La puissance crête diminue progressivement avec l'augmentation de la distance au point d'entrée de la fibre à décalage de fréquence 232 au fur et à mesure que la longueur d'onde centrale de l'impulsion se décale vers les hautes longueurs d'onde en raison du défaut quantique lié à l'interaction entre la lumière et la matière au travers de l'effet Raman.

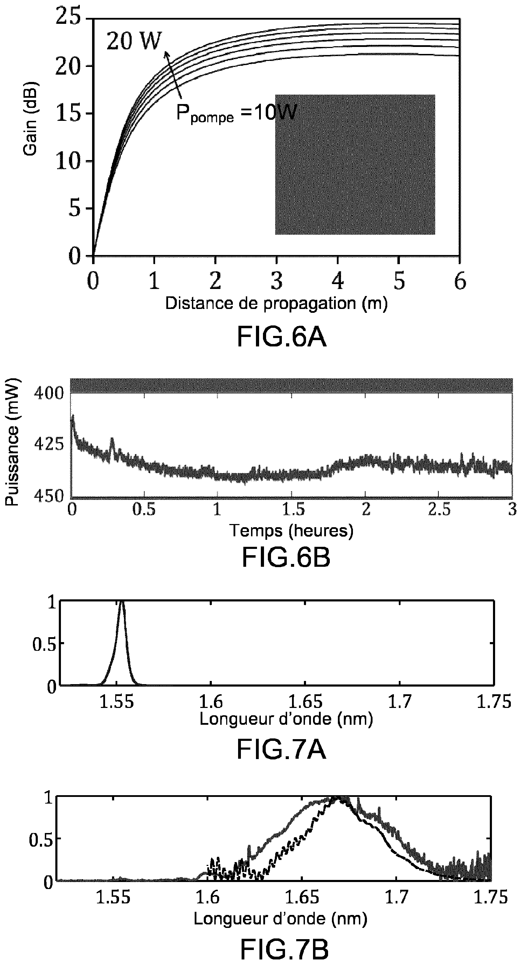

La FIG. 6A illustre la manière dont le gain en puissance augmente dans la fibre amplificatrice 229 en fonction de la distance par rapport au point d'entrée de cette fibre. On observe par exemple que, pour obtenir un gain de 20dB, il faut une longueur de fibre d'au-moins 2 m. Au-delà, l'augmentation du gain devient de moins en moins importante : elle suit une courbe asymptotique avec un maximum de gain autour de 25dB.

La FIG. 6B illustre la dérive de la puissance moyenne du train d'impulsions IL3 mesurée en sortie de la fibre à décalage de fréquence 232. On constate que la dérive en puissance est très faible, d'environ 10 à 15 mW pour une puissance moyenne de 425 mW, soit une dérive d'environ 2 à 3% sur une période d'utilisation de 3 heures. Cette très faible dérive est parfaitement compatible avec les applications dans le domaine biomédical précitées.

La FIG. 7A est un exemple de spectre de l'impulsion IL1, en fonction de la longueur d'onde $\lambda_1$ : ce spectre est centré sur la longueur d'onde $\lambda_1$ = 1555 nm. La FIG. 7B est un exemple de spectre de l'impulsion IL2 en fonction de la longueur d'onde $\lambda_2$ : ce spectre est centré sur la longueur d'onde $\lambda_2$ = 1675 nm, illustrant le décalage en longueur d'onde. On observe également par comparaison de la FIG. 7A avec la FIG. 7B, la compression temporelle subie par cette impulsion, correspondant sur cette figure à un élargissement du spectre.

La présente description concerne également un système d'imagerie comprenant un système laser selon l'un quelconque des modes de réalisation décrit dans ce document, un microscope pour former et acquérir une image, selon une technique de microscopie multiphotonique, à partir d'impulsions ultra-brèves produites au moyen d'une source laser selon la présente description. Les impulsions sont envoyées vers une zone à imager. Cette zone à imager est par exemple un tissu biologique. Les impulsions provoquent à une profondeur donnée dans le tissu biologique une excitation des molécules. Un faisceau optique émis en réponse par la zone à imager est détecté au sein du microscope. Une image peut être acquise à partir du faisceau optique détecté.

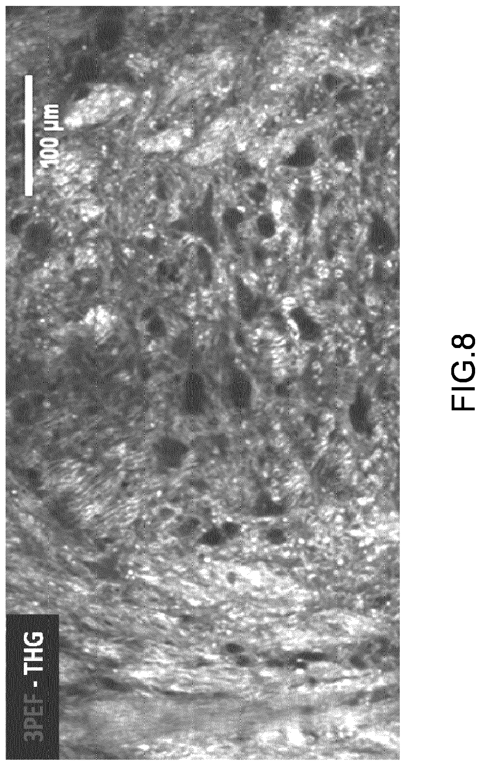

La FIG. 8 est un exemple d'image obtenue par un système d'imagerie selon la présente description. La figure illustre un exemple d'imagerie multi-contraste à trois photons basée sur la fluorescence par excitation à trois photons 3PEF (« 3-photon excited fluorescence », rouge) et la génération de troisième harmonique THG (« third harmonie génération » ou THG, blanc), réalisée avec la source laser développée sur un échantillon de tissu de tronc cérébral de souris transgénique Rainbow-3 (ce type de souris est décrit par exemple dans le document intitulé "Developmental bias in cleavage-stage mouse blastomeres,", par I. Tabansky et al, Current Biology, 2013). L'imagerie de troisième harmonique permet d'avoir une image structurelle sans marquage de cette zone du tronc cérébral. Le signal de fluorescence 3PEF provient d'un marquage du tissu avec une expression plus forte des marqueurs au niveau des vaisseaux sanguins.

D'autres applications à l'imagerie biologique peuvent être envisagées. Par exemple une source laser entièrement fibrée selon la présente description émettant à une longueur d'onde comprise entre 900 et 950 nm peut être utilisée pour la microscopie biphotonique lorsqu'une protéine fluorescente verte (GFP selon l'anglicisme « Green Fluorescent Protein ») est utilisée comme marqueur. Cette source pourra être construite à partir de fibres optiques amplificatrices dopées au néodyme.

D'autres applications pour une source laser entièrement fibrée selon la présente description sont envisageables lorsqu'on utilise le thulium ou l'holmium comme terre rare. Le rayonnement IL1 sera obtenu à une longueur d'onde de 1,9 à 2,1 $\mu$m tandis que le rayonnement à IL2 aura une longueur d'onde supérieure à 2,1 $\mu$m. Les impulsions ultra-brèves de forte puissance crête obtenues selon l'invention pourront être utilisées pour générer un rayonnement secondaire ultra-bref dans le domaine spectral UV par génération d'harmoniques d'ordre élevé.

Une source laser entièrement fibrée selon l'invention peut aussi être utilisée pour générer, par un doublage de fréquence dans un cristal non-linéaire approprié, des impulsions femtosecondes à une longueur d'onde moitié de celle issue du laser selon la présente description. Par exemple si le matériau de dopage est l'holmium, la longueur d'onde des impulsions IL1 et IL2 sera proche de 2150 nm tandis que la longueur d'onde des impulsions IL3 en sortie de la fibre à décalage de fréquence pourra être comprise entre 2200 et 2600 nm. Par un doublage de fréquence dans un cristal non-linéaire adéquat, la longueur d'onde des impulsions pourra être divisée par deux et atteindre la gamme entre 1100 et 1300 nm qui n'est actuellement pas couverte par les lasers femtoseconde à fibre de forte puissance crête. Les impulsions de forte puissance crête ainsi générées pourront être utilisées dans des applications d'imagerie biologique mettant en œuvre la THG et la 3PEF.

**Revendications**

1. Source laser femtoseconde comprenant :

   - un oscillateur laser d'injection (211) à fibre optique dopée dans un matériau donné, adapté pour délivrer, via une fibre optique de sortie (212), une première impulsion picoseconde, à une première longueur d'onde $\lambda_1$ ;
   - un amplificateur de puissance (220) à fibre optique amplificatrice (229) pour produire, à partir de la première impulsion, une deuxième impulsion, à la première longueur d'onde, avec une énergie amplifiée par rapport à la première impulsion, la fibre optique amplificatrice (229) étant dopée dans le même matériau que la fibre optique (212) de l'oscillateur d'injection, présentant une longueur inférieure ou égale à la distance du point de compression solitonique et supérieure à la distance à partir de laquelle la fibre optique amplificatrice (229) opère en régime non-linéaire;
   - une fibre à décalage de fréquence (232) adaptée pour recevoir la deuxième impulsion et générer par auto-décalage Raman un soliton fondamental à une deuxième longueur d'onde $\lambda_2$ strictement supérieure à la première longueur d'onde $\lambda_1$, ladite fibre à décalage présentant une aire modale supérieure à une aire modale de la fibre amplificatrice (229).

2. Source laser femtoseconde selon la revendication 1, dans laquelle la fibre optique amplificatrice (229) présente une aire modale supérieure ou égale à 200 $\lambda_1^2$.

3. Source laser femtoseconde selon l'une quelconque des revendications 1 à 2, dans lequel l'oscillateur laser d'injection est un oscillateur à fibres à gestion de dispersion chromatique configuré pour générer des impulsions à dérive de fréquence.

4. Source laser femtoseconde selon l'une quelconque des revendications 1 à 2, dans lequel l'oscillateur laser d'injection comprend un oscillateur solitonique délivrant des impulsions sans dérive de fréquence.

5. Source laser femtoseconde selon la revendication 3, dans laquelle l'oscillateur solitonique est suivi d'une fibre optique à dispersion normale configurée pour étirer temporellement les impulsions générées par l'oscillateur laser d'injection.

6. Source laser femtoseconde selon l'une quelconque des revendications précédentes, dans laquelle le matériau de dopage est choisi dans le groupe comprenant l'ytterbium, le praséodyme l'erbium, le thulium, l'holmium et le bismuth.

7. Source laser femtoseconde selon l'une quelconque des revendications précédentes, dans laquelle la fibre optique amplificatrice (229) et la fibre à décalage de fréquence (232) sont soudées l'une à l'autre.

8. Source laser femtoseconde selon l'une quelconque des revendications précédentes, comprenant en outre un filtre passe-haut (234) pour éliminer des résidus d'impulsion à la première longueur d'onde $\lambda_1$.

9. Système d'imagerie comprenant une source laser femtoseconde selon l'une quelconque des revendications précédentes, adaptée à l'émission d'impulsions vers un objet en profondeur dans un milieu biologique, et un microscope (250) pour former et acquérir une image de l'objet à partir de la lumière de fluorescence rétrodiffusée par l'objet.

10. Procédé de génération d'impulsions laser femtosecondes comprenant

    - une production, par un oscillateur d'injection à fibre optique dopée dans un matériau donné, d'une première impulsion picoseconde, à une première longueur d'onde $\lambda_1$;

- une production, par un amplificateur de puissance à fibre optique amplificatrice, à partir de la première impulsion, d'une deuxième impulsion, à la première longueur d'onde, avec une énergie amplifiée par rapport à la première impulsion, la fibre optique amplificatrice étant dopée dans le même matériau que la fibre optique de l'oscillateur d'injection, présentant une longueur inférieure ou égale à la distance du point de compression solitonique et supérieure à la distance à partir de laquelle la fibre optique amplificatrice opère en régime non-linéaire;

une production, par auto-décalage Raman, au moyen d'une fibre à décalage de fréquence adaptée pour recevoir la deuxième impulsion, d'un soliton fondamental à une deuxième longueur d'onde $\lambda_2$ strictement supérieure à la première longueur d'onde $\lambda_1$, ladite fibre à décalage présentant une aire modale supérieure à une aire modale de la fibre amplificatrice.

**Patentansprüche**

1. Femtosekundenlaserquelle, Folgendes beinhaltend:

   - einen Einspeise-Laseroszillator (211) mit mit einem gegebenen Material dotiertem Lichtwellenleiter, geeignet zum Abgeben, über einen Ausgangslichtwellenleiter (212), eines ersten Picosekundenimpulses mit einer ersten Wellenlänge $\lambda_1$;
   - einen Leistungsverstärker (220) mit Verstärker-Lichtwellenleiter (229) zum Erzeugen, anhand des ersten Impulses, eines zweiten Impulses, mit der ersten Wellenlänge, mit einer in Bezug auf den ersten Impuls verstärkten Energie, wobei der Verstärker-Lichtwellenleiter (229) mit demselben Material dotiert ist wie der Lichtwellenleiter (212) des Einspeise-Laseroszillators, aufweisend eine Wellenlänge kleiner als der oder gleich dem Abstand zum Soliton-Kompressionspunkt und größer als der Abstand, ab welchem der Verstärker-Lichtwellenleiter (229) im nichtlinearen Betrieb arbeitet;
   - einen Frequenzverschiebungs-Lichtwellenleiter (232), welcher dafür geeignet ist, den zweiten Impuls aufzunehmen und durch Raman-Auto-Verschiebung einen Grund-Soliton mit einer Wellenlänge $\lambda_2$ streng größer als die erste Wellenlänge $\lambda_1$ zu erzeugen, wobei der Verschiebungs-Lichtwellenleiter eine größere Modalfläche als eine Modalfläche des Verstärkungs-Lichtwellenleiters (229) aufweist.

2. Femtosekundenlaserquelle nach Anspruch 1, bei welcher der Verstärker-Lichtwellenleiter (229) eine Modalfläche größer oder gleich 200 $\lambda_1^2$ aufweist.

3. Femtosekundenlaserquelle nach einem der Ansprüche 1 bis 2, bei welcher der Einspeise-Laseroszillator ein Lichtwellenleiter-Oszillator mit gelenkter chromatischer Streuung ist, welcher konfiguriert ist, um Impulse mit Frequenzdrift zu erzeugen.

4. Femtosekundenlaserquelle nach einem der Ansprüche 1 bis 2, bei welcher der Einspeise-Laseroszillator einen Soliton-Oszillator beinhaltet, welcher Impulse ohne Frequenzdrift abgibt.

5. Femtosekundenlaserquelle nach Anspruch 3, bei welcher der Soliton-Oszillator von einem Lichtwellenleiter mit normaler Streuung gefolgt ist, um die von dem Einspeise-Laseroszillator erzeugten Impulse zeitlich zu verlängern.

6. Femtosekundenlaserquelle nach einem der vorhergehenden Ansprüche, bei welcher das Dotierungsmaterial aus der Gruppe gewählt ist, beinhaltend Ytterbium, Praseodym, Erbium, Thulium, Holmium und Bismut.

7. Femtosekundenlaserquelle nach einem der vorhergehenden Ansprüche, bei welcher der Verstärker-Lichtwellenleiter (229) und der Frequenzverschiebungs-Lichtwellenleiter (232) miteinander verschweißt sind.

8. Femtosekundenlaserquelle nach einem der vorhergehenden Ansprüche, zudem beinhaltend einen Hochpassfilter (234) zum Eliminieren der Impulsrückstände mit der ersten Wellenlänge $\lambda_1$.

9. Bildgebungssystem, beinhaltend eine Femtosekundenlaserquelle nach einem der vorhergehenden Ansprüche, zum Senden von Impulsen in Richtung eines Objekts in die Tiefe in einem biologischen Milieu geeignet, und ein Mikroskop (250) zum Bilden und Erfassen eines Bildes des Objekts anhand des durch das Objekt zurückgestreuten Fluoreszenzlichtes.

10. Verfahren zum Erzeugen von Femtosekundenlaserimpulsen, Folgendes beinhaltend:

- eine Erzeugung, durch einen Einspeise-Laseroszillator mit mit einem gegebenen Material dotiertem Lichtwellenleiter, eines ersten Picosekunden-Impulses mit einer ersten Wellenlänge $\lambda_1$;
- eine Erzeugung, durch einen Leistungsverstärker mit Verstärker-Lichtwellenleiter, anhand des ersten Impulses, eines zweiten Impulses, mit der ersten Wellenlänge, mit einer in Bezug auf den ersten Impuls verstärkten Energie, wobei der Verstärker-Lichtwellenleiter mit demselben Material dotiert ist wie der Lichtwellenleiter des Einspeise-Laseroszillators, aufweisend eine Wellenlänge kleiner als der oder gleich dem Abstand zum Soliton-Kompressionspunkt und größer als derjenige Abstand, ab welchem der Verstärker-Lichtwellenleiter im nichtlinearen Betrieb arbeitet;
- eine Erzeugung, durch Raman-Auto-Verschiebung, mit Hilfe eines Frequenzverschiebungs-Lichtwellenleiters, welcher dafür geeignet ist, den zweiten Impuls aufzunehmen, eines Grund-Solitons mit einer Wellenlänge $\lambda_2$ streng größer als die erste Wellenlänge $\lambda_1$, wobei der Verschiebungs-Lichtwellenleiter eine größere Modalfläche als eine Modalfläche des Verstärkungs-Lichtwellenleiters aufweist.

**Claims**

1. A femtosecond laser source comprising:

   - an injecting laser oscillator (211) based on optical fiber doped with a given dopant, suitable for delivering, via an exit optical fiber (212), a first picosecond pulse, at a first wavelength $\lambda_1$;
   - a power amplifier (220) based on amplifying optical fiber (229) for producing, from the first pulse, a second pulse, at the first wavelength, with an energy that is amplified with respect to the first pulse, the amplifying optical fiber (229) being doped with the same dopant as the optical fiber (212) of the injecting oscillator, having a length smaller than or equal to the distance of the soliton compression point and larger than the distance from which the amplifying optical fiber (229) operates in non-linear regime; and
   - a frequency-shifting fiber (232) suitable for receiving the second pulse and generating by Raman self-shifting a fundamental soliton at a second wavelength $\lambda_2$ strictly longer than the first wavelength $\lambda_1$, said shifting fiber having a modal area larger than an modal area of the amplifying fiber (229).

2. The femtosecond laser source as claimed in claim 1, wherein the amplifying optical fiber (229) has a modal area larger than or equal to $200\lambda_1^2$.

3. The femtosecond laser source as claimed in any one of claims 1 to 2, wherein the injecting laser oscillator is a fiber oscillator with chromatic dispersion management configured to generate frequency-chirped pulses.

4. The femtosecond laser source as claimed in any one of claims 1 to 2, wherein the injecting laser oscillator comprises a soliton oscillator that delivers pulses that are not frequency chirped.

5. The femtosecond laser source as claimed in claim 3, wherein the soliton oscillator is followed by a normal-dispersion optical fiber configured to temporally stretch the pulses generated by the injecting laser oscillator.

6. The femtosecond laser source as claimed in any one of the preceding claims, wherein the dopant is chosen from the group comprising ytterbium, praseodymium, erbium, thulium, holmium and bismuth.

7. The femtosecond laser source as claimed in any one of the preceding claims, wherein the amplifying optical fiber (229) and the frequency-shifting fiber (232) are spliced to each other.

8. The femtosecond laser source as claimed in any one of the preceding claims, furthermore comprising a high-pass filter (234) for removing pulse residues at the first wavelength $\lambda_1$.

9. An imaging system comprising a femtosecond laser source as claimed in any one of the preceding claims, suitable for emitting pulses toward an object located at depth in a biological medium, and a microscope (250) for forming and acquiring an image of the object from fluorescence light backscattered by the object.

10. A method for generating femtosecond laser pulses, comprising

   - producin, with an injecting oscillator based on optical fiber doped with a given dopant, a first picosecond pulse, at a first wavelength $\lambda_1$;

- producing, with a power amplifier based on amplifying optical fiber, from the first pulse, a second pulse, at the first wavelength, with an energy that is amplified with respect to the first pulse, the amplifying optical fiber being doped with the same dopant as the optical fiber of the injecting oscillator, having a length smaller than or equal to the distance of the soliton compression point and larger than the distance from which the amplifying optical fiber operates in non-linear regime;

- producing, by Raman self-shifting, by means of a frequency-shifting fiber suitable for receiving the second pulse, a fundamental soliton at a second wavelength $\lambda_2$ strictly longer than the first wavelength $\lambda_1$, said shifting fiber having a modal area larger than a modal area of the amplifying fiber.

ART ANTERIEUR

FIG.1

EP 3 491 703 B1

FIG.2A

FIG.2B

EP 3 491 703 B1

FIG.3A

FIG.3B

FIG.3C

EP 3 491 703 B1

FIG.4A

FIG.4B

FIG.4D

FIG.4C

FIG.4E

EP 3 491 703 B1

$\Delta\nu = c/\lambda_b - c/\lambda_a$

FIG.5A

$\Delta\nu = c/\lambda_b - c/\lambda_a$

FIG.5B

FIG.5C

EP 3 491 703 B1

FIG.6A

FIG.6B

FIG.7A

FIG.7B

FIG.8

**EP 3 491 703 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description

- **HORTON et al.** In vivo three-photon microscopy of subcortical structures within an intact mouse brain. *Nature Photonics,* 20 Janvier 2013
- **PATRICK CADROAS et al.** Three-Photon Microscopy with a Monolithic All-Fiber Format Laser Emitting at 1650 nm. *ADVANCED PHOTONICS,* 2016
- **WANG KE et al.** Tunable high-energy soliton pulse generation from a large-mode-area fiber and its application to third harmonie generation microscopy. *APPLIED PHYSICS LETTERS, US,* 15 Août 2011, vol. 99 (7), 71112-71112
- **LUC JEUNHOMME.** Single-mode fiber optics. Marcel Dekker, 1983, 99
- **I. TABANSKY et al.** Developmental bias in cleavage-stage mouse blastomeres. *Current Biology,* 2013